# FASCICULE DE BREVET EUROPEEN

(11) **EP 1 331 985 B1**
(45) Date de publication et mention de la délivrance du brevet: **08.06.2005**
(21) Numéro de dépôt: 01980622.3
(22) Date de dépôt: 22.10.2001
(51) Int. Cl.: B01F 3/08

(54) **PROCEDE DE PREPARATION D'UNE EMULSION DONT LA PHASE ORGANIQUE EST DE FORTE VISCOSITE**
VERFAHREN ZUR HERSTELLUNG EINER EMULSION, DEREN ORGANISCHE PHASE EINE HOHE VISKOSITÄT HAT
METHOD FOR PREPARING AN EMULSION WITH HIGH-VISCOSITY ORGANIC PHASE

(30) Priorité: 20.10.2000 FR 0013465
(43) Date de publication de la demande: 06.08.2003
(73) Titulaire: RHODIA CHIMIE, 92512 Boulogne Billancourt Cedex (FR)
(72) Inventeur: LANNIBOIS-DREAN, Hélène, F-94220 CHARENTON LE PONT (FR); MORVAN, Mikel, F-92600 ASNIERES (FR); LABEAU, Marie-Pierre, F-75019 Paris (FR); VIDIL, Christine, F-69360 COMMUNAY (FR)
(74) Mandataire: Delenne, Marc
(86) Numéro de dépôt international: PCT/FR2001/003273
(87) Numéro de publication internationale: WO 2002/032560

(56) Documents cités:
- WO-A-00/00528
- WO-A-00/35961
- WO-A-95/04774
- WO-A-97/00275
- WO-A-97/09400
- WO-A-98/29487
- US-A- 5 658 981
- DATABASE WPI Section Ch, Week 199407 Derwent Publications Ltd., London, GB; Class A14, AN 1994-054029 XP002174110 & JP 06 009848 A (SANYO CHEM IND LTD), 18 janvier 1994 (1994-01-18)

## Description

La présente invention a pour objet un procédé de préparation d'une émulsion dont la phase organique présente une viscosité élevée.

On rencontre des difficultés à préparer des émulsions présentant une taille de gouttelettes relativement faible, et obtenues à partir d'une phase continue aqueuse et une phase discontinue organique dont la viscosité est élevée, c'est-à-dire au moins égale à 1 Pa.s, de préférence au moins 5 Pa.s. En effet, la différence de viscosité entre les phases continue et discontinue fait qu'il est nécessaire de mettre en oeuvre des moyens très cisaillants et/ou peu productifs. En outre les résultats atteints ne sont que partiellement satisfaisants.

Des solutions ont été proposées pour obtenir des émulsions huile dans eau mettant en oeuvre des moyens classiques. Ces émulsions sont obtenues en mettant en jeu, en particulier, des quantités importantes de tensioactifs. Ainsi, on procède à partir d'une solution aqueuse concentrée de tensioactif, en pied de cuve, à laquelle on ajoute la phase organique. L'émulsion obtenue peut ensuite être diluée.

L'inconvénient de ce procédé est qu'il nécessite l'emploi d'un matériel spécialement conçu. De plus, ce type de procédé ne peut être mis en oeuvre en continu.

En outre, il n'est pas possible de préparer des émulsions multiples (eau dans huile dans eau) en mettant en jeu le procédé décrit ci-dessus. En effet, dès que l'on introduit l'émulsion inverse (eau dans huile) dans la phase aqueuse concentrée de tensioactifs, l'émulsion inverse est déstabilisée et l'on ne peut obtenir qu'une émulsion simple (huile dans eau).

La préparation des émulsions huile dans eau en utilisant un polymère thermoépaississant est divulguée dans les documents WO-A-97 00275 et WO-A-95 04774. Des polymères thermoépaississants sont décrits dans le document US-A-5 658 981.

La présente invention a pour objet d'obtenir un procédé simple et efficace de préparation d'une émulsion huile dans eau à partir d'une phase organique visqueuse, c'est-à-dire d'une part, ne nécessitant de mettre en oeuvre que des moyens classiques, et d'autre part, permettant d'obtenir une taille de particules pour l'émulsion, fine et régulière.

De plus, la présente invention est tout particulièrement adaptée à l'obtention d'émulsions multiples.

Ainsi, la présente invention a pour objet un procédé de préparation d'une émulsion huile dans eau, dont la phase organique présente une viscosité supérieure ou égale à 1 Pa.s dans lequel on met en oeuvre une phase aqueuse comprenant au moins un polymère thermoépaississant présentant un saut de viscosité entre 25 et 80°C tel que la valeur du rapport log₁₀ (viscosité à 80°C) / log₁₀ (viscosité à 25°C) est au moins égale à 1, de préférence au moins égale à 2, la variation de la viscosité étant réversible ; la quantité de polymère thermoépaississant étant telle que la viscosité de la phase aqueuse soit de 0,2 à 5 fois celle de la phase organique à la température de préparation de l'émulsion ; celle-ci étant supérieure ou égale à la température d'épaississement du polymère thermoépaississant.

Mais d'autres caractéristiques et avantages de la présente invention apparaîtront plus clairement à la lecture de la description et de l'exemple, qui vont suivre.

Tout d'abord, il est à noter que le procédé selon l'invention peut être mis en oeuvre pour l'obtention d'émulsions simples huile dans eau, mais aussi pour des émulsions multiples pour lesquelles la phase organique est en fait une émulsion inverse (donc eau dans huile).

Il est précisé que la taille moyenne des gouttelettes d'une émulsion simple obtenue selon l'invention, est plus particulièrement comprise entre 0,1 et 50 µm, de préférence entre 0,1 et 5 µm.

Dans le cas d'émulsions multiples obtenues selon l'invention, la taille moyenne des gouttelettes dispersées dans la phase aqueuse externe est comprise entre 5 et 100 µm, plus particulièrement entre 5 et 50 µm, avantageusement entre 5 et 15 µm.

Les tailles moyennes des gouttelettes sont mesurées au moyen d'un granulomètre Horiba, et correspondent au diamètre médian en volume (d₅₀) qui représente le diamètre de la particule égal à 50 % de la distribution cumulative.

Dans ce qui va suivre, le terme "émulsion" est utilisé indifféremment pour désigner une émulsion simple directe (huile dans eau), une émulsion inverse (eau dans huile) ou une émulsion multiple, à moins que la nature de l'émulsion ne soit indiquée précisément.

Dans ce qui va suivre, il sera fait référence à la "phase aqueuse interne" pour désigner la phase aqueuse de l'émulsion inverse de l'émulsion multiple. Le terme "phase aqueuse" désignera indifféremment la phase aqueuse d'une émulsion simple directe, ou la phase aqueuse "externe" d'une émulsion multiple.

Par ailleurs, le terme "polymères" désigne à la fois des homopolymères et des copolymères.

La phase organique va maintenant être décrite.

Tout d'abord, le composé utilisé en tant que phase organique est plus particulièrement choisi parmi les composés dont la solubilité dans l'eau ne dépasse pas 10 % en poids à 25°C.

En outre, comme cela a été indiqué auparavant, la phase organique présente une viscosité d'au moins 1 Pa.s, de préférence d'au moins 5 Pa.s. Le procédé selon l'invention est tout particulièrement approprié pour la préparation d'émulsions pour lesquelles la phase organique présente une viscosité comprise entre 5 et 500 Pa.s.

Notons que la viscosité fait référence à la viscosité dynamique, mesurée à 25°C à l'aide d'un viscosimètre Brookfield selon la norme AFNOR NFT 76 102 de février 1972. La phase organique est plus particulièrement choisie parmi les huiles minérales ; les résines alkydes (comme par exemple les résines Coporob 3115 DE, commercialisées par la société Novance) ; les polyisocyanates ; les silicones de haut poids moléculaire ; ces composés étant seuls ou en mélange.

Parmi les huiles minérales on peut citer les huiles polybutènes. Par exemple, conviennent les huilespolybutènes obtenues par polymérisation de la coupe C dont la proportion en isobutène est élevée (gammes Napvis, Hyvis de BP).

Pour ce qui a trait aux polyisocyanates, on peut citer notamment les composés de formule suivante : A(-NCObloc)ₚ, où A représente un squelette organique présentant n valences libres, p étant compris entre 2 et 7, NCObloc représente une fonction isocyanate masquée ou non.

Plus particulièrement, le nombre total de carbones dudit monomère est avantageusement compris entre 10 et 100.

En outre, le squelette A peut être constitué à partir d'une polyamine lourde, y compris les anilines, par exemple présentant un nombre de carbone au moins égal à 6, plus particulièrement au moins égal à 10, de préférence au moins égal à 15. Cette amine est transformée de manière connue en soi par réaction avec du phosgène. Il est à noter que le squelette A peut également être celui des trimères et des biurets.

Parmi les groupes masquant, on peut choisir les groupements à hydrogène mobile dont le pKa est au plus égal à 14, plus particulièrement au plus égal à 10, de préférence au plus égal à 8. Il est à noter que plus le pKa est élevé, plus il est souhaitable que l'agent masquant, s'il est présent, soit volatil.

Les agents masquant sont choisis de manière que l'émulsion soit stable à sa température de stockage.

Parmi les fonctions chimiques susceptibles de masquer les isocyanates, on peut citer à titre d'exemples, les fonctions suivantes :
- les alcools et les thiols
- les oximes
- les hydroxylamines
- les acides
- les amides et les imides
- les β-dicétones
- les pyrazoles.

Les polyisocyanates convenables sont plus particulièrement choisis parmi les huiles et/ou gommes et/ou résines à groupements (poly)isocyanates dont la viscosité se trouve dans les gammes indiquées auparavant. On ne sortirait pas du cadre de l'invention en utilisant plusieurs composés de ce type, de même que leur association avec au moins un solvant (ou diluant) de ladite huile et/ou gomme et/ou résine, dès l'instant que la viscosité de l'ensemble est dans la gamme indiquée.

Parmi les silicones de haut poids moléculaire, on peut citer par exemple les huiles et/ou gommes et/ou résines polyorganosiloxaniques. On ne sortirait pas du cadre de la présente invention en mettant en oeuvre des mélanges d'huile(s) et/ou de gomme(s) et/ou de résine(s) polyorganosiloxanique(s), dès l'instant que les mélanges présentent une viscosité dans les gammes indiquées précédemment. De même, l'invention est appropriée pour émulsionner des mélanges d'huile(s) et/ou de gomme(s) et/ou de résine(s) polyorganosiloxanique(s), et éventuellement d'au moins un solvant desdites huile(s) et/ou gomme(s) et/ou résine(s), et/ou éventuellement d'au moins un silane et/ou d'au moins une charge siliceuse ou non siliceuse, dès l'instant que les mélanges présentent des viscosités dans le domaine mentionné.

Parmi les huiles et les gommes polyorganosiloxaniques pouvant être mises en oeuvre, on peut citer celles constituées des motifs de formule

R'₃₋ₐ Rₐ Si O_{1/2} et R₂ Si O

formules où :
- a est un entier de 0 à 3
- les radicaux R, identiques ou non, représentent un radical aliphatique, saturé ou non, en C₁-C₁₀ ; un radical aromatique en C₆-C₁₃ ; un groupe organique polaire lié au silicium par un liaison Si-C ou Si-O-C ; un atome d'hydrogène ;
- les radicaux R', identiques ou non, représentent un groupe OH ; un radical alcoxy ou alcényloxy en C₁-C₁₀ ; un radical aryloxy en C₆-C₁₃ ; un radical acyloxy en C₁-C₁₃ ; un radical cétiminoxy en C₁-C₈ ; un radical amino- ou amido-fonctionnel en C₁-C₆, lié au silicium par une liaison Si-N.

De préférence au moins 80 % des radicaux R desdites huiles représentant un groupe méthyle.

Parmi les résines polyorganosiloxaniques pouvant être mises en oeuvre, on peut citer celles constituées de motifs de formules :

R Si O _{3/2} (motif T) et/ou Si O₂ (motif Q)

associés à des motifs de formules :

R'₃₋ₐ Rₐ Si O_{1/2} (motif M) et/ou R₂ Si O (motif D)

formules dans lesquelles a, R et R' ont la définition donnée ci-dessus.

Celles-ci sont généralement du type MQ, MDQ, TDM, TD, MT .....

Lorsque lesdites huiles, gommes ou résines contiennent des radicaux R réactifs et/ou polaires, tels que H, OH, vinyle, allyle, héxényle, aminoalkyles notamment, ces derniers ne représentent généralement pas plus de 2 % du poids de l'huile ou de gomme et pas plus de 10 % du poids de la résine.

Les huiles polydiméthylsiloxanes et α,ω-bis(hydroxy)polydiméthylsiloxanes visqueuses ainsi que les gommes polydiméthylsiloxanes, polyphénylméthylsiloxane et α,ω-bis(hydroxy)polydiméthylsiloxanes sont des produits du commerce bien connus.

Les résines visqueuses polyméthylsiloxanes DT contenant de 1 à 2 % en poids de fonctions silanols sont également des produits du commerce.

Parmi les solvants des huiles, gommes ou résines silicones, éventuellement présents dans la phase silicone, on peut citer les organopolysiloxanes cycliques volatils (octaméthylcyclotétrasiloxane, decaméthylcyclopentasiloxane, ...), les huiles polydiméthylsiloxanes courtes (viscosité inférieure à 100 mPa.s.), l'hexaméthyldisiloxane, les cétones (méthyléthylcétone ...) les éthers (éther diéthylique ...), les esters (myristate d'isopropyle, acétate d'éthyle, ......), certains solvants chlorés ou chlorofluorés (chlorure de méthylène, chloroforme ...), les paraffines très ramifiées (huiles blanches à base d'isoalcanes et de cycloalcanes ...).

Des silanes et/ ou des charges minérales diverses peuvent de même être présents dans la phase silicone.

Ces silanes peuvent être notamment des sous-produits de synthèse ou des agents de réticulation desdites huiles, gommes ou résines polyorganosiloxaniques. Ils sont généralement présents selon des quantités de l'ordre de 0 à 10 parties en poids, de préférence de l'ordre de 0 à 5 parties en poids pour 100 parties en poids d'huile(s) et/ou de gomme(s) et/ou de résine polyorganosiloxanique lorsqu'il s'agit de sous-produits réactionnels.

Lorsque leur fonction d'agent de réticulation des huiles, gommes ou résines hydroxylées est recherchée, ils sont généralement présents selon des quantités de l'ordre de 0,5 à 30 parties en poids, de préférence de l'ordre de 2 à 8 parties en poids pour 100 parties en poids d'huile(s) et/ ou de gomme(s) et/ou de résine(s).

Lesdits silanes peuvent aussi être un additif permettant de moduler les propriétés physico-chimiques, d'adhérence notamment des compositions silicones d'applications diverses obtenues à partir des émulsions aqueuses préparées selon le procédé de l'invention. Parmi cette catégorie de silanes on peut citer l'aminopropyltriéthoxysilane, l'aminopropylméthyldiéthoxysilane, le glycidoxypropyl-triméthoxysilane ... Ils sont mis en oeuvre selon des quantités pouvant aller jusqu'à 200 %, généralement de l'ordre de 2 à 100 % du poids d'huile(s) et/ou de gomme(s) et/ ou résine(s).

Des charges siliceuses ou non siliceuses renforçantes ou semi-renforçantes peuvent aussi être présentes. A titre d'exemple, on peut citer les silices colloïdales, les poudres de silice de combustion et de précipitation, les terres de diatomées, le quartz broyé, le carbonate de calcium naturel, l'alumine hydratée, l'hydroxyde de magnésium, le noir de carbone, le dioxyde de titane, l'oxyde d'aluminium, la vermiculite, l'oxyde de zinc, le mica, le talc, l'oxyde de fer, le sulfate de baryum, la chaux éteinte ...

La taille de ces charges est généralement inférieure ou égale à la taille moyenne des gouttelettes dans lesquelles elles sont dispersées. A titre indicatif, la taille moyenne de ces charges (d₅₀) est généralement de l'ordre de 0,001 à 50 µm, de préférence de l'ordre de 0,001 à 10 µm.

Les charges sont généralement présentes selon des quantités pouvant aller jusqu'à 300 %, de préférence de l'ordre de 3 à 100 % du poids d'huile(s) et/ou de gomme(s) et/ou de résine(s).

La phase organique peut de même être choisie parmi les résines époxy, les huiles essentielles, les mono-, di- et tri- glycérides, dès l'instant que leur viscosité est dans la gamme indiquée auparavant.

La phase organique peut éventuellement comprendre au moins une matière active hydrophobe.

Il est à noter que la phase organique elle-même peut constituer la matière active hydrophobe dès l'instant que la phase organique présente une viscosité dynamique dans la gamme indiquée précédemment.

Dans le cas où elle est différente de la phase organique, la matière active se présentent sous forme liquide ou non, soluble dans la phase organique ou solubilisée dans un solvant organique miscible à ladite phase organique, ou encore sous la forme d'un solide dispersé dans ladite phase.

Plus particulièrement, les matières actives sont telles que leur solubilité dans l'eau ne dépasse pas 10 % en poids, à 25°C.

En outre, les matières actives présentent de préférence une température de fusion inférieure ou égale à 100°C, plus particulièrement inférieure ou égale à 80°C.

A titre d'exemple de matières actives dans le domaine de l'alimentaire, on peut citer les mono-, di- et triglycérides, les huiles essentielles, les arômes, les colorants.

A titre d'exemple de matières actives dans le domaine de la cosmétique on peut citer les huiles silicones appartenant par exemple à la famille des diméthicones ; les vitamines lipophiles comme la vitamine A.

A titre d'exemple de matières actives convenables pour la réalisation de l'invention, dans le domaine des peintures, on peut citer les résines alkydes, les résines époxy, les isocyanates masqués ou non.

Dans le domaine du papier, on peut citer à titre d'exemple les résines de collage et d'hydrofugation telles que le dimère d'alkylcétène (AKD) ou l'anhydride alcényle succinique (ASA).

Dans le domaine de l'agrochimie, les matières actives phytosanitaires peuvent être choisies parmi la famille des α-cyano-phénoxybenzyl carboxylates ou des α-cyano-halogénophénoxy-carboxylates, la famille des N-méthylcarbonates comprenant des substituants aromatiques, les matières actives telles que Aldrin, Azinphos-methyl, Benfluralin, Bifenthrin, Chlorphoxim, Chlorpyrifos, Fluchloralin, Fluroxypyr, Dichlorvos, Malathion, Molinate, Parathion, Permethrin, Profenofos, Propiconazole, Prothiofos, Pyrifenox, Butachlor, Metolachlor, Chlorimephos, Diazinon, Fluazifop-P-butyl, Heptopargil, Mecarbam, Propargite, Prosulfocarb, Bromophos-ethyl, Carbophenothion, Cyhalothrin.

Dans le domaine de la détergence, on peut mentionner en tant que matière active possible les antimousses silicones.

Il est de même possible d'utiliser des matières actives telles que celles entrant dans la composition de lubrifiants pour le travail ou la déformation des matériaux. La matière active est habituellement une huile, un dérivé d'une huile ou encore un ester d'acide gras ou un sel d'acide gras.

La matière active peut aussi être choisie parmi les solvants organiques ou les mélanges de tels solvants pas ou peu miscibles dans l'eau, comme notamment ceux mis en oeuvre pour le nettoyage ou le décapage, tels que les coupes pétrolières aromatiques, les composés terpéniques comme les D- ou L- limonènes, ainsi que les solvants comme le Solvesso®. Conviennent aussi comme solvants, les esters aliphatiques, comme les esters méthyliques d'un mélange d'acides acétique, succinique et glutarique (mélange d'acides sous-produit de la synthèse du Nylon), les huiles comme l'huile de vaseline, et les solvants chlorés.

Au cas où la phase organique comprend une ou plusieurs matières actives hydrophobes différentes de la phase organique, leur teneur représente plus particulièrement 1 à 50 % en poids de ladite phase organique.

Selon une variante de la présente invention, la phase organique comprend une phase aqueuse interne dispersée. Plus particulièrement, la phase organique est une émulsion inverse.

Notons que la viscosité de l'émulsion inverse, c'est-à-dire de la phase organique comprenant la phase aqueuse interne, présente, elle aussi, une viscosité importante. Ainsi, la viscosité de l'émulsion inverse est d'au moins 1 Pa.s, plus particulièrement d'au moins 5 Pa.s et de préférence comprise entre 5 et 500 Pa.s. Les viscosités mentionnées ci-dessus sont des viscosités dynamiques mesurées au Brookfield à 25°C, selon la norme NFT 76 102 de février 1972.

Dans un tel cas, la phase organique de l'émulsion inverse est de préférence de la même nature que celle d'une émulsion simple. On pourra donc se référer en tout point à la liste donnée ci-dessus.

La phase aqueuse interne, si elle est présente, et/ou la phase aqueuse externe de l'émulsion peuvent comprendre au moins une matière active hydrophile. De préférence, la matière active hydrophile se trouve dans la phase aqueuse interne de l'émulsion, lorsque celle-ci est présente.

Il est précisé que les matières actives hydrophobes et hydrophiles sont déterminées en fonction de leur compatibilité entre elles. De même, la matière active hydrophile est choisie de manière à ne pas interférer sur la phase organique.

La matière active hydrophile peut se présenter sous une forme soluble dans la phase aqueuse ; sous une forme solubilisée dans un solvant miscible à l'eau comme le méthanol, l'éthanol, le propylène glycol, le glycérol, par exemple ; ou encore sous la forme d'un solide en dispersion dans ladite phase.

A titre d'exemple de matières actives utilisables dans le domaine de la cosmétique, on peut citer les substances qui ont un effet cosmétique, un effet thérapeutique ou tout autre substance utilisable pour le traitement de la peau et du cheveu.

Ainsi, on peut utiliser, en tant que matière active des agents conditionneurs de la peau et du cheveu, comme notamment les polymères comprenant des ammonium quaternaires qui peuvent éventuellement être engagés dans des hétérocycles (composés du type des quaternium, polyquaternium, etc.)., des agents humectants ; des agents fixants (styling) qui sont plus particulièrement choisis parmi des polymères (homo-, co- ou ter-polymères par exemple acrylamide, acrylamide/acrylate de sodium, polystyrène sulfonate, etc.), les polymères cationiques, la polyvinylpyrrolidone, l'acétate de polyvinyle,etc.

Il est de même possible d'utiliser des agents colorants ; des agents astringents, utilisables dans les déodorants et qui sont plus particulièrement des sels d'aluminium, de zirconium ; des agents antibactériens ; des agents anti-inflammatoires, des agents anesthésiants, des filtres solaires, etc.

On peut aussi citer les α- et β- hydroxyacides, comme les acides citrique, lactique, glycolique, salicylique ; les acides dicarboxyliques de préférence insaturés et comprenant 9 à 16 atomes de carbone comme l'acide azélaique ; la vitamine C et ses dérivés, notamment les dérivés glycosylés et phosphatés ; les biocides notamment cationiques (notamment Glokill PQ, Rhodaquat RP50, commercialisés par Rhodia Chimie).

Dans le domaine de l'alimentaire, on peut citer par exemple les sels divalents de calcium (les phosphates, les chlorures, etc.) utilisés comme agent réticulant de polymères texturant comme les alginates, les carraghénannes ; le bicarbonate de sodium, entre autres.

Dans le domaine des matières actives phytosanitaires, on peut utiliser des pesticides hydrophiles ou des éléments nutritifs hydrophiles favorisant la croissance et le développement des plantes.

Pour ce qui a trait au domaine de l'exploitation ou la construction de puits de gisement de pétrole ou de gaz, la présente invention peut être mise en oeuvre pour des matières actives hydrophiles utilisables notamment lors des opérations cimentation, complétion, forage et stimulation des puits (par exemple la fracturation). A titre d'exemples de matières actives utilisables dans ce domaine, on peut mentionner des catalyseurs de réticulation de compositions cimentaires, comme par exemple les sels de lithium, comme le chlorure, l'acétate. On peut de même citer des composés susceptibles, entre autres, de dégrader les polysaccharides, comme par exemple les acides carboxyliques (notamment l'acide citrique), des enzymes (notamment les cellulases), des oxydants.

Dans le domaine des silicones, on peut citer par exemple les sels de calcium, la potasse utilisés comme agent réticulant.

Dans le domaine de la fabrication du papier, on peut citer notamment le chlorure de calcium, l'acide chlorhydrique.

La quantité de matière active hydrophile est plus particulièrement comprise entre 0,1 et 50 % en poids par rapport à la phase aqueuse (que celle-ci soit interne et/ou externe), de préférence comprise entre 0,1 et 20 % en poids par rapport à la phase aqueuse (interne et/ou externe).

Dans le cas où une phase aqueuse interne est présente, le rapport pondéral phase aqueuse interne / phase organique est plus particulièrement compris entre 10/90 et 90/10. De préférence, ce rapport pondéral est compris entre 30/70 et 80/20.

Toujours selon cette variante, c'est-à-dire celle selon laquelle la phase organique se présente sous la forme d'une émulsion inverse, l'émulsion inverse comprend en outre au moins un tensioactif non ionique et/ou au moins un polymère amphiphile à blocs, et/ou au moins un tensioactif cationique.

Selon une première variante, l'émulsion inverse comprend au moins un tensioactif non ionique ou au moins un polymère amphiphile à blocs, ou un mélange d'entre eux.

Il est à noter que la règle de Bancroft peut être appliquée au tensioactif non ionique et au polymère amphiphile à blocs utilisés (2^{ème} Congrès Mondial de l'Emulsion, 1997, Bordeaux, France). En d'autres termes, la fraction soluble dans la phase continue est supérieure à la fraction soluble dans la phase dispersée.

Ainsi, le tensioactif et le polymère sont de préférence choisis parmi ceux qui vérifient à la fois les deux conditions ci-dessous :
- lorsqu'ils sont mélangés avec la phase organique interne, à une concentration comprise entre 0,1 et 10 % en poids de ladite phase à 25°C, se trouvent sous la forme d'une solution dans tout ou partie de la gamme de concentration indiquée.
- lorsqu'ils sont mélangés avec la phase aqueuse interne, à une concentration comprise entre 0,1 et 10 % en poids de ladite phase et à 25°C, se trouvent sous la forme d'une dispersion dans tout ou partie de la gamme de concentration indiquée.

Plus particulièrement, le tensioactif non ionique est choisi parmi les composés présentant une valeur de HLB (balance hydrophile/lipophile) inférieure ou égale à 8.

A titre d'exemples de tensioactifs susceptibles d'entrer dans la composition de l'émulsion inverse, on peut citer les tensioactifs, seuls ou en mélange, choisis parmi :
- les alcools gras alcoxylés
- les triglycérides alcoxylés
- les acides gras alcoxylés
- les esters de sorbitan éventuellement alcoxylés
- les amines grasses alcoxylées
- les di(phényl-1 éthyl) phénols alcoxylés
- les tri(phényl-1 éthyl) phénols alcoxylés
- les alkyls phénols alcoxylés
le nombre de motifs alcoxylés (éthoxylés, propoxylés, butoxylés) est tel que la valeur de HLB soit inférieure ou égale à 8.

Les alcools gras alcoxylés comprennent généralement de 6 à 22 atomes de carbone, les motifs alcoxylés étant exclus de ces nombres.

Les triglycérides alcoxylés peuvent être des triglycérides d'origine végétale ou animale.

Les esters de sorbitan éventuellement alcoxylés sont des esters du sorbitol cyclisés d'acide gras comprenant de 10 à 20 atomes de carbone comme l'acide laurique, l'acide stéarique ou l'acide oléïque.

Les amines grasses alcoxylées ont généralement de 10 à 22 atomes de carbone, les motifs alcoxylés étant exclus de ces nombres.

Les alkylphénols alcoxylés ont généralement un ou deux groupes alkyles, linéaires ou ramifiés, ayant 4 à 12 atomes de carbone. A titre d'exemple on peut citer notamment les groupes octyle, nonyle ou dodécyle.

Quant au polymère amphiphile à blocs, celui-ci comprend au moins deux blocs.

Ces polymères amphiphiles, vérifiant la règle de Bancroft et les deux conditions énoncées auparavant, comprennent plus particulièrement au moins un bloc hydrophobe et au moins un bloc hydrophile neutre, anionique ou cationique.

Au cas où le polymère amphiphile comprend au moins trois blocs, et plus particulièrement trois blocs, le polymère est de préférence linéaire: En outre, les blocs hydrophobes se trouvent plus particulièrement aux extrémités.

Au cas où les polymères comprennent plus de trois blocs, ces derniers sont de préférence sous la forme de polymères greffés ou peignes.

Dans ce qui suit, même si cela constitue un abus de langage, le terme polymère amphiphile à blocs sera utilisé indifféremment pour les polymères linéaires à blocs et les polymères greffés ou peignes.

Lesdits polymères amphiphiles peuvent de manière avantageuse, être obtenus par polymérisation radicalaire dite vivante ou contrôlée. A titre d'exemples non limitatifs de procédés de polymérisation dite vivante ou contrôlée, on peut notamment se référer aux demandes WO 98/58974 (xanthate), WO 97/01478 (dithioesters), WO 99/03894 (nitroxydes) ; WO 99/31144 (dithiocarbamates).

Les polymères amphiphiles peuvent aussi être obtenus par polymérisation cationique, anionique.

Ils peuvent de même être préparés en mettant en jeu des polymérisations par ouverture de cycle (notamment anionique ou cationique), ou par modification chimique du polymère.

Les polymères greffés ou peignes peuvent encore être obtenus par des méthodes dites de greffage direct et copolymérisation.

Le greffage direct consiste à polymériser le(s) monomère(s) choisi(s) par voie radicalaire, en présence du polymère sélectionné pour former le squelette du produit final. Si le couple monomère/squelette ainsi que les conditions opératoires, sont judicieusement choisis, alors il peut y avoir réaction de transfert entre le macroradical en croissance et le squelette. Cette réaction génère un radical sur le squelette et c'est à partir de ce radical que croît le greffon. Le radical primaire issu de l'amorceur peut également contribuer aux réactions de transfert.

Pour ce qui a trait à la copolymérisation, elle met en oeuvre dans un premier temps le greffage à l'extrémité du futur segment pendant, d'une fonction polymérisable par voie radicalaire. Ce greffage peut être réalisé par des méthodes usuelles de chimie organique. Puis, dans un second temps, le macromonomère ainsi obtenu est polymérisé avec le monomère choisi pour former le squelette et on obtient un polymère dit "peigne".

Parmi les monomères hydrophobes à partir desquels le ou les blocs hydrophobes du polymère amphiphile peuvent être préparés, on peut citer notamment :
- les esters des acides mono- ou poly- carboxyliques, linéaires, ramifiés, cycliques ou aromatiques, comprenant au moins une insaturation éthylénique,
- les esters d'acides carboxyliques saturés comprenant 8 à 30 atomes de carbone, éventuellement porteurs d'un groupement hydroxyle ;
- les nitriles αβ-éthyléniquement insaturés, les éthers vinyliques, les esters vinyliques, les monomères vinylaromatiques, les halogénures de vinyle ou de vinylidène,
- les monomères hydrocarbonés, linéaires ou ramifiés, aromatiques ou non, comprenant au moins une insaturation éthylénique,
- les monomères de type siloxane cyclique ou non, les chlorosilanes ;
- l'oxyde de propylène, l'oxyde de butylène ;
seuls ou en mélanges, ainsi que les macromonomères dérivant de tels monomères.

A titre d'exemples particuliers de monomères hydrophobes susceptibles d'entrer dans la préparation du ou des blocs hydrophobes du polymère amphiphile à blocs, on peut citer :
- les esters d'acide (méth)acrylique avec un alcool comprenant 1 à 12 atomes de carbone comme le (méth)acrylate de méthyle, le (méth)acrylate d'éthyle, le (méth)acrylate de propyle, le (méth)acrylate de n-butyle, le (méth)acrylate de t-butyle, le (méth)acrylate d'isobutyle, l'acrylate de 2-éthylhexyl ;
- l'acétate de vinyle, le Versatate® de vinyle, le propionate de vinyle, le chlorure de vinyle, le chlorure de vinylidène, le méthyl vinyléther, l'éthyl vinyléther ;
- les nitriles vinyliques incluent plus particulièrement ceux ayant de 3 à 12 atomes de carbone, comme en particulier l'acrylonitrile et le méthacrylonitrile ;
- le styrène, l'α-méthylstyrène, le vinyltoluène, le butadiène, le chloroprène ;
seuls ou en mélanges, ainsi que les macromonomères dérivant de tels monomères.

Les monomères préférés sont les esters de l'acide acryliques avec les alcools linéaires ou ramifiés en C₁-C₄ tels que l'acrylate de méthyle, d'éthyle, de propyle et de butyle, les esters vinyliques comme l'acétate de vinyle, le styrène, l'α-méthylstyrène.

En ce qui concerne les monomères hydrophiles non ioniques à partir desquels les polymères amphiphiles à blocs peuvent être obtenus, on peut mentionner, sans intention de s'y limiter, l'oxyde d'éthylène, les amides des acides mono- ou poly-carboxyliques, linéaires, ramifiés, cycliques ou aromatiques, comprenant au moins une insaturation éthylénique ou dérivés, comme le (méth)acrylamide, le N-méthylol (méth)acrylamide ; les esters hydrophiles dérivant de l'acide (méth)acrylique comme par exemple le (méth)acrylate de 2-hydroxyéthyle ; les esters vinyliques permettant d'obtenir des blocs alcool polyvinylique après hydrolyse, comme l'acétate de vinyle, le Versatate® de vinyle, le propionate de vinyle, seuls, en combinaison, ainsi que les macromonomères dérivant de tels monomères. Il est rappelé que le terme macromonomère désigne une macromolécule portant une ou plusieurs fonctions polymérisables.

Toutefois les monomères hydrophiles préférés sont l'acrylamide et le méthacrylamide, seuls ou en mélange, ou sous la forme de macromonomères.

En ce qui concerne les monomères hydrophiles anioniques à partir desquels les polymères amphiphiles à blocs peuvent être obtenus, on peut mentionner, par exemple les monomères comprenant au moins une fonction carboxylique, sulfonique, sulfurique, phosphonique, phosphorique, sulfosuccinique, ou les sels correspondants.

Il est précisé que dans les conditions de pH d'utilisation du polymère amphiphile à blocs, les fonctions du ou des blocs anioniques du polymère se trouvent sous une forme au moins partiellement ionisée (dissociée). Plus particulièrement, au moins 10 % en mole des fonctions du ou des blocs sont sous forme ionisée. La détermination de cette valeur ne pose pas de problème à l'homme de l'art ; elle est notamment fonction du pKa des fonctions ionisables des motifs du polymère et du nombre de ces fonctions (soit du nombre de moles de monomère portant des fonctions ionisables mis en oeuvre lors de la préparation du polymère).

Plus particulièrement, les monomères sont choisis parmi :
- les acides mono- ou poly- carboxyliques linéaires, ramifiés, cycliques ou aromatiques, les dérivés N-substitués de tels acides ; les monoesters d'acides polycarboxyliques, comprenant au moins une insaturation éthylénique ;
- les acides vinyl carboxyliques linéaires, ramifiés, cycliques ou aromatiques ;
- les aminoacides comprenant une ou plusieurs insaturations éthyléniques ;
seuls ou en mélanges, leurs précurseurs, leurs dérivés sulfoniques ou phosphoniques, ainsi que les macromonomères dérivant de tels monomères ; les monomères ou macromonomères pouvant être sous la forme de sels.

A titre d'exemples de monomères anioniques, on peut citer sans intention de s'y limiter :
- l'acide acrylique, l'acide méthacrylique, l'acide fumarique, l'acide itaconique, l'acide citraconique, l'acide maléique, l'acide acrylamido glycolique, l'acide 2-propène 1-sulfonique, l'acide méthallyl sulfonique, l'acide styrène sulfonique, l'acide α-acrylamido méthylpropane sulfonique, le 2-sulfoéthylène méthacylate, l'acide sulfopropyl acrylique, l'acide bis-sulfopropyl acrylique, l'acide bis-sulfopropyl méthacrylique, l'acide sulfatoéthyl méthacrylique, le monoester phosphate d'acide hydroxyéthyl méthacrylique, ainsi que les sels de métal alcalin, comme le sodium, le potassium, ou d'ammonium ;
- l'acide vinyl sulfonique, l'acide vinylbenzène sulfonique, l'acide vinyl phosphonique, l'acide vinylidène phosphorique, l'acide vinyl benzoïque, ainsi que les sels de métal alcalin, comme le sodium, le potassium, ou d'ammonium ;
- le N-méthacryloyl alanine, le N-acryloyl-hydroxy-glycine ;
seuls ou en mélanges, ainsi que les macromonomères dérivant de tels monomères.

On ne sortirait pas du cadre de la présente invention en mettant en oeuvre des monomères précurseurs de ceux qui viennent d'être cités. En d'autres termes, ces monomères présentent des motifs qui, une fois incorporés dans la chaîne polymère, peuvent être transformés, notamment par un traitement chimique tel que l'hydrolyse, pour redonner les espèces anioniques précitées. Par exemple, les monomères totalement ou partiellement estérifiés des monomères précités peuvent être mis en oeuvre pour être, par la suite, hydrolysés totalement ou en partie.

En tant que monomères hydrophiles cationiques à partir desquels les polymères amphiphiles à blocs peuvent être obtenus, on peut citer notamment :
- les (méth)acrylates d'aminoalkyle, les (méth)acrylamides d'aminoalkyle ;
- les monomères comprenant au moins une fonction amine secondaire, tertiaire ou quaternaire, ou un groupe hétérocyclique contenant un atome d'azote, la vinylamine, l'éthylène imine ;
- les sels d'ammonium de diallyldialkyl ;
seuls ou en mélanges, ou les sels correspondants, ainsi que les macromonomères dérivant de tels monomères.

Lesdits monomères peuvent présenter un contre-ion choisi parmi les halogénures comme par exemple le chlore, les sulfates, les hydrosulfates, les alkylsulfates (par exemple comprenant 1 à 6 atomes de carbone), les phosphates, les citrates, les formates, les acétates.

A titre d'exemples de monomères cationiques convenables figurent entre autres les monomères suivants :
- diméthyl amino éthyl (méth)acrylate, diméthyl amino propyl (méth)acrylate le ditertiobutyl aminoéthyl (méth)acrylate, le diméthyl amino méthyl (méth)acrylamide, le diméthyl amino propyl (méth)acrylamide;
- l'éthylène imine, la vinylamine, la 2-vinylpyridine, la 4-vinylpyridine ;
- le chlorure de triméthylammonium éthyl (méth)acrylate, le méthyl sulfate de triméthylammonium éthyl acrylate, le chlorure de benzyl diméthylammonium éthyl (méth)acrylate, le chlorure de 4-benzoylbenzyl diméthyl ammonium éthyl acrylate, le chlorure de triméthyl ammonium éthyl (méth)acrylamido, le chlorure de triméthyl ammonium de vinylbenzyle ;
- le chlorure d'ammonium de diallyldiméthyl ;
seuls ou en mélanges, ou leurs sels correspondants, ainsi que les macromonomères dérivant de tels monomères.

De préférence, les polymères amphiphiles à blocs présentent une masse molaire en poids inférieure ou égale à 100000 g/mol, plus particulièrement comprise entre 1000 et 50000 g/mol, de préférence entre 1000 et 20000 g/mol. Il est précisé que les masses molaires en poids indiquées ci-dessus sont des masses molaires théoriques, évaluées en fonction des quantités respectives des monomères introduites lors de la préparation desdits polymères.

De préférence, on met en oeuvre un polymère amphiphile à bloc de type non ionique.

A titre d'exemple de polymère amphiphile à blocs convenant à la mise en oeuvre de l'invention, on peut citer les polymères triblocs polyhydroxystéarate - polyéthylène glycol - polyhydroxystéarate (les produits de la gamme Arlacel de ICI en sont un exemple), les polymères à blocs polydiméthylsiloxane greffé polyéther polyalkyle (comme les produits de la marque Tegopren commercialisé par Goldschmidt).

Selon une deuxième variante, l'émulsion inverse comprend au moins un tensioactif cationique.

Dans le cas de cette variante, il est indiqué que le tensioactif cationique ne vérifie pas la règle de Bancroft énoncée auparavant. En effet, le tensioactif cationique est soluble dans la phase dispersée et non dans la phase continue de l'émulsion inverse.

Parmi les tensioactifs cationiques convenables, on peut notamment utiliser les amines grasses aliphatiques ou aromatiques, les amides gras aliphatiques, les dérivés d'ammonium quaternaire (Rhodaquat RP50 de Rhodia Chimie).

Enfin, une troisième variante de l'invention consiste à combiner les deux possibilités qui viennent d'être détaillées.

Quelle que soit la variante retenue, la teneur totale en tensioactif non ionique, en polymère amphiphile à blocs et/ou en tensioactif cationique représente plus particulièrement de 0,1 à 10 % en poids, de préférence de 2 à 10 % en poids par rapport à la phase aqueuse interne.

Conformément à un mode de réalisation particulièrement avantageux, dans la cas où la phase organique se trouve sous la forme d'une émulsion inverse, la phase aqueuse interne peut comprendre au moins un additif choisi parmi les sels tels que les halogénures de métaux alcalins ou alcalino-terreux (comme le chlorure de sodium, le chlorure de calcium), ou les sulfates de métaux alcalin ou alcalino-terreux (comme le sulfate de calcium), ou leurs mélanges. La phase aqueuse interne peut aussi comprendre, en tant qu'additif, au moins un sucre, comme le glucose par exemple, ou encore au moins un polysaccharide, comme notamment le dextran, ou leurs mélanges.

La concentration en sel dans la phase aqueuse interne, lorsque ce dernier est présent, est plus particulièrement comprise entre 0,05 et 1 mol/l, de préférence 0,1 à 0,4 mol/l.

La concentration en sucre et/ou polysaccharide est telle que la pression osmotique de la phase aqueuse interne comprenant ledit sucre et/ou polysaccharide, correspond à la pression osmotique d'une phase aqueuse interne comprenant 0,05 à 1 mol/l de sel.

Selon une caractéristique importante de l'invention, la phase aqueuse de l'émulsion comprend au moins un polymère thermoépaississant.

Les polymères thermoépaississants possèdent la particularité de donner des solutions aqueuses dont la viscosité augmente lorsque la température dépasse la température d'épaississement du polymère thermoépaississant ; température au delà de laquelle la viscosité du milieu dans lequel est présent ledit polymère augmente.

Plus particulièrement, ces polymères sont solubles dans l'eau à température ambiante, et au-delà de la température d'épaississement, une partie du polymère devient hydrophobe (partie thermosensible) : le polymère forme ainsi un réseau physique à l'échelle microscopique, ce qui se traduit à l'échelle macroscopique par une augmentation de la viscosité.

Ainsi que cela a été indiqué auparavant, les polymères thermoépaississants utilisés dans le procédé selon l'invention sont choisis parmi ceux présentant un saut de viscosité entre 25 et 80°C tel que la valeur du rapport log₁₀ (viscosité à 80°C) /log₁₀ (viscosité à 25°C) est au moins égale à au moins 1.

Le rapport est mesuré dans les conditions suivantes :
* Le polymère est tout d'abord mis en solution dans l'eau (extrait sec de 4 %).
* Le profil rhéologique est ensuite mesuré en mode écoulement à contrainte imposée, en effectuant un balayage de température entre 20°C et 80°C. La configuration utilisée est la géométrie cône-plan 4cm/1degré. La contrainte introduite dans le programme est choisie (en mode manuel) de telle sorte que le gradient à 25°C soit de 10 s⁻¹.
* La grandeur qui a été retenue pour caractériser le pouvoir thermoépaississant du polymère, soit le rapport log₁₀ (viscosité à 80°C) / log₁₀ (viscosité à 25°C), représente le saut de viscosité, exprimé en décades, de 25 à 80°C. Cette grandeur exprime en d'autres termes que la viscosité du milieu à 80°C est supérieure de 10ⁿ fois la viscosité du milieu à 25°C ; avec n entier compris entre 0 et 5.

Selon l'invention, le polymère thermoépaississant présente un saut de viscosité d'au moins une décade, de préférence d'au moins deux décades.

Outre cette caractéristique, les polymères thermoépaississants sont choisis de telle sorte que la variation de viscosité est réversible. En d'autres termes, la viscosité diminue lorsque la température diminue.

Parmi les polymères thermoépaississants utilisables, on peut citer les polysaccharides modifiés hydrophobes comme les carboxyméthyl celluloses, les méthyl celluloses, les hydroxyéthyl celluloses, les hydroxypropyl celluloses.

Dans le cas de ce type de polymères, il peut être avantageux de les mettre en oeuvre associés à au moins un tensioactif supplémentaire, choisi parmi les tensioactifs non ioniques ou encore anioniques.

Conviennent aussi les polymères synthétiques comme les polymères à base de N-isopropyl acrylamide, les polymères à base de N,N-diméthyl aminoéthyl méthacrylate.

Selon un mode de réalisation particulier de l'invention, les polymères thermoépaississants mis en oeuvre possèdent une structure peigne constituée d'un segment squelette polymérique sur lequel sont greffés au moins deux segments latéraux polymériques, identiques ou non, pour lequel soit le squelette polymérique, soit les segments latéraux polymériques, possèdent une température critique inférieure de solubilité, LCST, comprise entre 25 et 80°C.

Il est à noter que les termes segments couvrent aussi bien un enchaînement linéaire que ramifié.

Selon une première variante de l'invention, c'est le segment squelette polymérique qui possède une LCST comprise entre 25 et 80°C .

Selon une seconde variante de l'invention, qui est la préférée, ce sont les segments latéraux polymériques qui possèdent une LCST comprise entre 25 et 80°C .

Enfin, selon une autre variante de l'invention, on met en oeuvre plusieurs polymères agencés entre eux de manière à former une structure réticulée dans laquelle leurs segments polymériques possédant la LCST figurent les noeuds de réticulation et au moins une partie de leurs segments ne possédant pas de température critique inférieure de solubilité entre 25 et 80°C, établissent des connexions entre lesdits noeuds.

Selon ces variantes, le segment ne possédant pas la LCST requise, à savoir comprise entre 25 et 80°C, est pour sa part hydrosoluble au moins dans ce domaine de températures, de préférence entre 10 et 100°C.

En ce qui concerne le segment polymérique ne possédant pas de température critique LCST, il s'agit plus préférentiellement d'un polymère de type éthylénique hydrosoluble.

Ces polymères hydrosolubles peuvent être issus de la polymérisation de monomères éthyléniques hydrosolubles. Ces monomères peuvent être en particulier de type vinylique, acrylique, styrénique, diénique ou encore de type ester vinylique.

A titre d'exemple de monomères vinyliques, on peut citer l'acide vinyl sulfonique, l'acide méthallyl sulfonique ou leurs sels.

A titre d'exemples de monomères acryliques, on peut citer l'acide (méth)acrylique, les diacides tels que l'acide fumarique, l'acide itaconique, ou leurs sels, l'anhydride maléique, l'acrylamide et ses dérivés tels que l'acide acrylamido méthyl propane sulfonique ou leurs sels.

A titre d'exemples de monomères styréniques, on peut citer l'acide styrène sulfonique, l'acide vinyl benzoïque ou leurs sels.

Aux monomères hydrosolubles évoqués ci-dessus, on peut également associer ou leur substituer des monomères hydrophobes dont les motifs, une fois incorporés dans la chaîne polymère, peuvent être transformés, notamment par un traitement chimique tel que l'hydrolyse, en motifs hydrosolubles. Il s'agit par exemple de (méth)acrylate de méthyle, de (méth)acrylate de terbutyle, de (méth)acrylate de glycidyle et d'acétate de vinyle.

Enfin, des monomères organosolubles quelconques peuvent également être utilisés et incorporés dans la chaîne polymère sous forme de motifs hydrophobes. Présents en faibles quantités, dans le segment polymérique, ils permettent de contrôler la solubilité dans l'eau du polymère correspondant.

Bien entendu, les différents monomères sont sélectionnés de manière à ce que le segment polymérique correspondant présente une solubilité en milieu aqueux conforme à l'invention. Cet ajustement des quantités relatives en monomères correspondants relève des compétences de l'homme de l'art.

Sont notamment préférés selon l'invention, les monomères comme les acides acrylique ou méthacrylique, les acrylamides et leurs dérivés, les acides fumarique et maléïque et les monomères sulfonés tels que l'acide 2-acrylamide-méthyl-propane sulfonique et ses sels alcalins et le vinylsulfonate.

Plus préférentiellement, ce type de segment polymérique possède un poids moléculaire au moins supérieur à 1000 g/mol, et de préférence au moins supérieur à 20000 g/mol (mesuré par GPC aqueuse, étalon : POE).

D'une manière avantageuse, ces segments polymériques sont issus de la polymérisation de l'acide acrylique et/ou l'acide 2-acrylamide-méthyl-propane sulfonique.

En ce qui concerne les segments polymériques possédant une LCST comprise entre 25 et 80°C, ils dérivent de polymères polyoxyalkylènes.

Selon un mode préféré de l'invention, le ou les différents motifs oxyalkylène présents dans le polymère polyoxyalkylène possèdent au plus 6 atomes de carbone.

D'une manière préférentielle, les segments présentant une LCST sont constitués de motifs oxyéthylène (OE) et/ou de motifs oxypropylène (OP).

Les motifs OE et OP peuvent être agencés dans le segment thermosensible polymérique sous forme statistique, bloc ou séquencée. Le segment thermosensible polymérique peut par exemple présenter une structure étoilée. Il s'avère possible d'ajuster la température critique de solubilité à travers notamment la longueur et la composition de ces segments polymériques. De préférence, les segments présentant une température critique conforme à l'invention sont constitués d'au moins 5 motifs oxyalkylène.

Plus préférentiellement, il s'agit de macromonomères correspondants.

Au sens de la présente invention, un macromonomère désigne une macromolécule portant une ou plusieurs fonctions éthyléniques polymérisables par voie radicalaire.

Le greffage des segments polymériques latéraux sur un segment polymérique squelette peut être effectué selon des techniques classiques et familières à l'homme de l'art (European Polymer Journal 4, 343 (1968) par exemple).

Parmi ces techniques classiques, on peut notamment citer celles dites de greffage direct et copolymérisation.

Le greffage direct consiste à polymériser le(s) monomère(s) choisi(s) par voie radicalaire, en présence du polymère sélectionné pour former le squelette du produit final. Si le couple monomère/squelette ainsi que les conditions opératoires, sont judicieusement choisis, alors il peut y avoir réaction de transfert entre le macroradical en croissance et le squelette. Cette réaction génère un radical sur le squelette et c'est à partir de ce radical que croît le greffon. Le radical primaire issu de l'amorceur peut également contribuer aux réactions de transfert.

Pour sa part, la copolymérisation met en oeuvre dans un premier temps le greffage à l'extrémité du segment thermosensible d'une fonction polymérisable par voie radicalaire. Ce greffage peut être réalisé par des méthodes usuelles de chimie organique. Puis, dans un second temps, le macromonomère ainsi obtenu est polymérisé avec le monomère choisi pour former le squelette et on obtient un polymère dit "peigne". Il est évident pour l'homme de l'art que lorsqu'on polymérise un macromonomère et un monomère choisis de telle sorte que ces deux espèces s'associent fortement par liaisons hydrogène, alors il y a simultanément greffage direct sur le segment polymérique du macromonomère et incorporation de ce macromonomère dans la chaîne polymère par simple polymérisation de son extrémité polymérisable. Dans ce cas, la structure obtenue est sensiblement plus branchée ou même réticulée que dans les deux cas précédents.

De préférence, le polymère comprend 0,1% à 50 % molaire et de préférence 0,1 à 5,0 % molaire de segments polymériques possédant une LCST comprise entre 25 et 80°C.

Conviennent tout particulièrement à l'invention, les polymères thermoépaississants comprenant au moins :
- polymère préparé à partir de tri-blocs POE-POP-POE et d'acide acrylique (pourcentages molaires respectifs : 2,3 %, 97,7 %), de préférence par greffage direct,
- polymère préparé à partir de macromonomère de tri-blocs POE-POP-POE et d'acide acrylique (% molaires respectifs : 1,6 %, 98,4 %), de préférence par copolymérisation,
- polymère préparé à partir de macromonomère de tri-blocs POE-POP-POE et d'acide acrylique (% molaires respectifs : 3 %, 97 %), de préférence par copolymérisation, et/ou
- polymère préparé à partir de macromonomère de tri-blocs POE-POP-POE et d'acide acrylique (% molaires respectifs : 2 %, 98 %), de préférence par copolymérisation.

Ces polymères ont notamment été décrits dans la demande de brevet français FR 2 780 422.

La teneur en polymère thermoépaississant dans la phase aqueuse est telle que la viscosité de la phase aqueuse soit de 0,2 à 5 fois celle de la phase organique, de préférence, de 0,5 à 2 fois celle de la phase organique à la température de préparation de l'émulsion ; celle-ci étant supérieure ou égale à la température d'épaississement du polymère thermoépaississant.

A titre indicatif, la teneur en polymère thermoépaississant est plus particulièrement comprise entre 0,5 et 5 % en poids par rapport à la phase aqueuse. De préférence, la teneur en polymère thermoépaississant est comprise entre 1 et 3 % en poids par rapport à la phase aqueuse.

La phase aqueuse comprend en outre, de manière avantageuse :
- au moins un tensioactif non ionique et/ou au moins un polymère amphiphile non ionique éventuellement associé(s) à au moins un tensioactif anionique et/ou au moins un polymère amphiphile anionique ; la teneur totale en tensioactif(s) /polymère(s) amphiphile(s) non ioniques et anioniques est comprise entre 0,5 et 10 % en poids, de préférence entre 1 et 5 % en poids, par rapport à la phase organique ou à l'émulsion inverse si elle est présente ; la quantité en tensioactif et/ou polymère amphiphile anioniques représente 0,5 à 5 % en poids, de préférence 0,5 à 2 % en poids, par rapport au poids de tensioactif / polymère amphiphile non ioniques ; ou
- au moins un polymère amphiphile anionique éventuellement associé à au moins un tensioactif anionique ; la teneur totale en polymère amphiphile / tensioactif anioniques est comprise entre 0,5 et 10 % en poids, de préférence entre 1 et 5 % en poids, par rapport à la phase organique ou à l'émulsion inverse si elle est présente.

En ce qui concerne les tensioactifs non ioniques, on met en oeuvre de préférence des tensioactifs non ioniques polyalcoxylés.

De manière avantageuse, ledit tensioactif non ionique est choisi parmi les tensioactifs suivants, seuls ou en mélange :
- les alcools gras alcoxylés
- les triglycérides alcoxylés
- les acides gras alcoxylés
- les esters de sorbitan alcoxylés
- les amines grasses alcoxylées
- les di(phényl-1 éthyl) phénols alcoxylés
- les tri(phényl-1 éthyl) phénols alcoxylés
- les alkyls phénols alcoxylés
le nombre de motifs alcoxylés, plus particulièrement éthoxylés et/ou propoxylés, est tel que la valeur de HLB soit supérieure ou égale à 10.

Pour ce qui a trait au polymère amphiphile non ionique polyalcoxylé, ce dernier vérifie la règle de Bancroft et ses deux conditions énoncées auparavant, et comprend au moins deux blocs, l'un d'eux étant hydrophile, l'autre hydrophobe ; l'un au moins des blocs comprenant des motifs polyalcoxylés, plus particulièrement polyéthoxylés et/ou polypropoxylés.

Ce qui a été indiqué auparavant dans le cadre de la description des monomères hydrophiles non ioniques et des monomères hydrophobes utilisables pour la préparation des polymères amphiphiles à blocs entrant dans la composition de l'émulsion inverse, reste valable et ne sera pas repris ici.

A titre purement indicatif, lesdits polymères sont obtenus en mettant en jeu des polymérisations par ouverture de cycle, notamment anionique.

Plus particulièrement lesdits polymères amphiphiles polyalcoxylés non ioniques sont choisis parmi les polymères dont la masse molaire en poids est inférieure ou égale à 100000 g/mol (mesurée par GPC, étalon polyéthylène glycol), de préférence comprise entre 1000 et 50000 g/mol, de préférence comprise entre 1000 et 20000 g/mol.

A titre d'exemples de polymères de ce type, on peut citer entre autres les polymères triblocs polyéthylène glycol / polypropylène glycol / polyéthylène glycol. De tels polymères sont bien connus et sont notamment commercialisés sous les marques Pluronic (commercialisé par BASF), Arlatone (commercialisé par ICI).

Selon un autre mode de réalisation, le polymère amphiphile non ionique est un polymère amphiphile à blocs, obtenu par polymérisation d'au moins un monomère hydrophile non ionique et d'au moins un monomère hydrophobe, la proportion et la nature desdits monomères étant telles que le polymère résultant vérifie les conditions énoncées auparavant (règle de Bancroft - deux conditions).

Ces polymères amphiphiles comprennent de plus au moins un bloc hydrophobe et au moins un bloc hydrophile neutre (non ionique).

Au cas où ledit polymère comprend au moins trois blocs, et plus particulièrement trois blocs, le polymère est avantageusement linéaire. En outre, les blocs hydrophiles se trouvent plus particulièrement aux extrémités.

Au cas où les polymères comprennent plus de trois blocs, ces derniers sont de préférence sous la forme de polymères greffés ou peignes.

Les listes des monomères hydrophiles non ioniques, des monomères hydrophobes non ioniques, de même que les diverses méthodes de préparation, citées dans le cadre de la description des polymères amphiphiles à blocs, peuvent être reprise dans le cas des polymères selon cette variante.

Toutefois les monomères hydrophiles préférés sont l'acrylamide et le méthacrylamide, seuls ou en mélange, ou sous la forme de macromonomères ; les monomères préférés sont les esters de l'acide acryliques avec les alcools linéaires ou ramifiés en C₁-C₄ tels que l'acrylate de méthyle, d'éthyle, de propyle et de butyle, les esters vinyliques comme l'acétate de vinyle, le styrène, l'α-méthylstyrène.

Parmi les tensioactifs anioniques convenables, on peut citer entre autres, seuls ou en mélanges :
- les alkylesters sulfonates, par exemple de formule R-CH(SO₃M)-COOR', où R représente un radical alkyle en C₈-C₂₀, de préférence en C₁₀-C₁₆, R' un radical alkyle en C₁-C₆, de préférence en C₁-C₃ et M un cation alcalin (sodium, potassium, lithium), ammonium substitué ou non substitué (méthyl-, diméthyl-, triméthyl-, tétraméthylammonium, diméthylpipéridinium ...) ou dérivé d'une alcanolamine (monoéthanolamine, diéthanolamine, triéthanolamine ...). On peut citer tout particulièrement les méthyl ester sulfonates dont le radical R est en C₁₄-C₁₆ ; les alkylbenzènesulfonates, plus particulièrement en C₉-C₂₀, les alkylsulfonates primaires ou secondaires, notamment en C₈-C₂₂, les alkylglycérol sulfonates, les acides polycarboxyliques sulfonés, comme par exemple ceux décrits dans GB 1082179, les sulfonates de paraffine ;
- les alkylsulfates par exemple de formule ROSO₃M, où R représente un radical alkyle ou hydroxyalkyle en C₁₀-C₂₄, de préférence en C₁₂-C₂₀ ; M représentant un atome d'hydrogène ou un cation de même définition que ci-dessus, ainsi que leurs dérivés polyalcoxylés (éthoxylés (OE), propoxylés (OP), ou leurs combinaisons), comme par exemple le dodécylsulfate de sodium ;
- les alkyléthersulfates par exemple de formule RO(CH₂CH₂O)ₙSO₃M où R représente un radical alkyle ou hydroxyalkyle en C₁₀-C₂₄, de préférence en C₁₂-C₂₀ ; M représentant un atome d'hydrogène ou un cation de même définition que ci-dessus, n variant généralement de 1 à 4, ainsi que leurs dérivés polyalcoxylés (éthoxylés (OE), propoxylés (OP), ou leurs combinaisons), comme par exemple le laurylethersulfate avec n = 2.
- les alkylamides sulfates, par exemple de formule RCONHR'OSO₃M où R représente un radical alkyle en C₂-C₂₂, de préférence en C₆-C₂₀, R' un radical alkyle en C₂-C₃, M représentant un atome d'hydrogène ou un cation de même définition que ci-dessus, ainsi que leurs dérivés polyalcoxylés (éthoxylés (OE), propoxylés (OP), ou leurs combinaisons) ;
- les sels d'acides gras saturés ou insaturés, par exemple comme ceux en C₈-C₂₄, de préférence en C₁₄-C₂₀, les N-acyl N-alkyltaurates, les alkyliséthionates, les alkylsuccinamates et alkylsulfosuccinates, les monoesters ou diesters de sulfosuccinates, les N-acyl sarcosinates, les polyéthoxycarboxylates ; et
- les phosphates esters d'alkyle et/ou d'alkyléther et/ou d'alkylaryléther.

Parmi les polymères anioniques susceptibles d'être employés, on peut citer tout particulièrement les polymères à blocs, de préférence diblocs ou triblocs, obtenus par polymérisation d'au moins un monomère hydrophile anionique, éventuellement d'au moins un monomère hydrophile non ionique, et d'au moins un monomère hydrophobe.

Là encore, le choix des monomères et leurs proportions respectives sont telles que le polymère résultant vérifie les deux conditions précédemment énoncées (règle de Bancroft).

Les monomères hydrophiles non ioniques, anioniques, les monomères hydrophobes ainsi que les modes de synthèse cités dans le cadre de la description des polymères amphiphiles entrant dans la composition d'émulsions pour lesquelles la phase continue est une phase huile, peuvent être mis en oeuvre pour l'obtention des polymères selon cette variante. On pourra donc s'y référer.

Selon un mode de réalisation particulier, et toujours dans le cas de la variante selon laquelle la phase organique se présente sous la forme d'une émulsion inverse, il peut être avantageux d'ajouter dans la phase aqueuse au moins un additif choisi parmi les sels tels que les halogénures de métaux alcalins, ou alcalino-terreux (comme le chlorure de sodium, le chlorure de calcium), au moins un sulfate de métal alcalin ou alcalino-terreux (comme le sulfate de calcium), au moins un sucre (glucose par exemple), ou encore au moins un polysaccharide (notamment le dextran) ou leurs mélanges.

L'ajout de ce type d'additif permet d'équilibrer, si nécessaire, les pressions osmotiques de la phase aqueuse de l'émulsion et de la phase aqueuse interne (de l'émulsion inverse) ; les concentrations en sel, en sucre et/ou en polysaccharide sont fixées dans ce sens.

De plus, selon l'application à laquelle est destinée l'émulsion selon l'invention, ou selon la nature de la ou des matières actives employées, il peut être souhaitable d'ajuster le pH de la phase aqueuse externe par ajout de base (soude, potasse) ou d'acide (chlorhydrique).

Selon une variante avantageuse de la présente invention, la phase aqueuse de l'émulsion peut comprendre au moins un polymère épaississant. Ce polymère a pour effet d'éviter le crémage et/ou la sédimentation de l'émulsion finale.

A titre d'illustration, on peut utiliser des polymères épaississants extraits de végétaux et éventuellement modifiés, tels que les carraghénannes, les alginates, les carboxyméthyl celluloses, les méthylcelluloses, les hydroxypropyl celluloses, les hydroxyéthyl celluloses.

On peut de même employer des polymères épaississants du type des polysaccharides d'origine animale, végétale, bactérienne, on peut citer à titre d'exemple non limitatif, la gomme xanthane, le guar et dérivés (tels que l'hydroxypropyl guar par exemple), les polydextroses, ou leurs combinaisons. ,

Lorsqu'il est présent, la teneur en polymère épaississant est plus particulièrement comprise entre 0,1 et 2 % en poids par rapport à la phase aqueuse, de préférence entre 0,1 et 0,5 % en poids par rapport à la phase aqueuse. Précisons que dans cette gamme de concentration, le polymère épaississant est soluble dans la phase aqueuse.

Le rapport pondéral phase organique / phase aqueuse, ou le rapport pondéral de l'ensemble phase aqueuse interne et phase organique / phase aqueuse est habituellement compris entre 10/90 et 90/10, de préférence compris entre 30/70 et 80/20.

Le procédé de préparation de l'émulsion consiste plus particulièrement à mélanger sous agitation :
+ la phase organique, comprenant :
   - éventuellement au moins une matière active hydrophobe,
   - éventuellement la phase aqueuse interne dispersée comprenant éventuellement au moins une matière active hydrophile et éventuellement au moins un additif ; l'ensemble phase aqueuse interne et phase organique comprenant au moins un tensioactif non ionique et/ou au moins un polymère amphiphile à blocs, et/ou au moins un tensioactif cationique ; et
+ la phase aqueuse comprenant :
   - éventuellement au moins une matière active hydrophile,
   - au moins un tensioactif non ionique polyalcoxylé et/ou au moins un polymère amphiphile non ionique et/ou au moins tensioactif anionique et/ou au moins un polymère amphiphile anionique,
   - au moins un polymère thermoépaississant,
   - éventuellement au moins un additif et éventuellement au moins un polymère épaississant ;
+ la température de préparation de l'émulsion étant supérieure ou égale à la température d'épaississement du polymère thermoépaississant.

Il est de même précisé que la température de préparation de l'émulsion est de préférence supérieure ou égale à la température de fusion de la phase organique.

De manière avantageuse, l'appareillage mis en oeuvre pour l'agitation est tout à fait classique dans le domaine. Ainsi, on peut utiliser une pale cadre.

Il est à noter que l'agitation est plutôt lente, de l'ordre de 300 à 700 tours/minute.

Dans le cas où la phase organique est constituée par une émulsion inverse, le procédé consiste à préparer tout d'abord l'émulsion inverse puis à mélanger cette dernière avec la phase aqueuse.

Les préparations d'une émulsion simple directe (huile dans eau) ou d'une émulsion multiple peuvent être réalisées, elles aussi, selon toute méthode connue.

Ainsi, à titre d'exemple de préparation d'une simple directe, on prépare d'une part, un premier mélange comprenant le composé constituant la phase organique interne et éventuellement la matière active hydrophobe.

D'autre part, on prépare un deuxième mélange comprenant l'eau, le tensioactif et/ou le polymère amphiphile non ioniques et/ou anioniques, le polymère thermoépaississant, éventuellement la matière active et/ou l'additif (sel, sucre et/ou polysaccharide) et/ou le polymère épaississant. Selon un mode de réalisation particulier de préparation d'émulsion simple directe, la phase aqueuse ne comprend pas d'additif ni de polymère épaississant.

La préparation de la phase aqueuse consiste de préférence à mélanger tout d'abord l'eau et la matière active si elle est utilisée, puis le tensioactif et/ou le polymère amphiphile, sous agitation. Puis on ajoute le polymère thermoépaississant,

Cette opération a lieu en général à une température comprise entre 25 et 80°C, de préférence comprise entre 40 et 70°C. Il n'est pas nécessaire que la température lors du mélange des divers composés de la phase aqueuse soit supérieure ou égale égale à la température d'épaississement du polymère thermoépaississant.

L'émulsion est ensuite obtenue en ajoutant la phase organique à la phase aqueuse, sous agitation. II est précisé à nouveau que selon une caractéristique de l'invention, cette opération a lieu à une température supérieure ou égale à la température d'épaississement du polymère thermoépaississant, de manière à limiter la différence de viscosité entre les deux phases de l'émulsion.

Plus particulièrement, cette opération est réalisée à une température d'au moins 25°C, plus particulièrement comprise entre 25 et 80°C, de préférence entre 40 et 70°C.

Il est possible de mettre en oeuvre une étape d'affinage de l'émulsion directe.

La durée de l'agitation peut être déterminée sans difficulté par l'homme de l'art et dépend du type d'appareillage mis en oeuvre. Elle est de préférence suffisante pour obtenir une taille moyenne de gouttelettes (dso) comprise dans les gammes indiquées auparavant.

Dans le cas d'une émulsion multiple, un exemple de préparation consiste à préparer un premier mélange constituant la phase aqueuse interne, comprenant l'eau, éventuellement la matière active hydrophile, le tensioactif cationique s'il est présent, et éventuellement l'additif (sel, sucre et/ou polysaccharide). Un deuxième mélange est aussi préparé, comprenant le composé constituant la phase organique interne, éventuellement la matière active hydrophobe et le tensioactif et/ou le polymère amphiphile à blocs non ioniques, s'ils sont présents.

On ajoute ensuite le premier mélange au second, sous agitation.

La préparation de l'émulsion inverse est en général réalisée à une température supérieure à la température de fusion du composé constituant la phase organique interne. Plus particulièrement, la température de préparation de l'émulsion inverse est comprise entre 20 et 80°C. De manière avantageuse, la température de préparation de l'émulsion inverse est voisine de la température de préparation de l'émulsion multiple.

Il est possible de mettre en oeuvre une étape d'affinage de l'émulsion inverse.

La durée de l'agitation peut être déterminée sans difficulté par l'homme de l'art et dépend du type d'appareillage mis en oeuvre. Elle est de préférence suffisante pour obtenir une taille moyenne de gouttelettes (d₅₀) comprise dans les gammes indiquées auparavant.

On prépare ensuite la phase aqueuse externe de l'émulsion. Ceci peut notamment être réalisé en mélangeant le tensioactif et/ou le polymère amphiphile non ioniques et/ou anioniques, le polymère thermoépaississant, éventuellement la matière active et/ou l'additif et/ou le polymère épaississant et l'eau. De préférence, on mélange tout d'abord l'eau et la matière active, l'additif s'ils sont présents, le tensioactif et/ou le polymère amphiphile, sous agitation. Puis on ajoute le polymère thermoépaississant, et le cas échéant, le polymère épaississant. Précisons que selon un mode de réalisation tout à fait avantageux, le polymère épaississant s'il est employé, n'est ajouté qu'une fois l'émulsion multiple obtenue. Dans ce cas, il est utilisé sous la forme d'une solution aqueuse. La teneur en eau étant telle que les gammes de concentration de l'émulsion multiple sont vérifiées.

Cette opération a en général lieu à une température comprise entre 25 et 80°C, de préférence comprise entre 40 et 70°C. Il n'est pas nécessaire que la température lors du mélange des divers composés de la phase aqueuse externe soit supérieure ou égale à la température d'épaississement du polymère thermoépaississant.

On procède ensuite à la préparation de l'émulsion multiple proprement dite en ajoutant l'émulsion inverse à la phase aqueuse qui ont été préparées auparavant.

Cette opération a lieu à une température supérieure ou égale à la température d'épaississement du polymère thermoépaississant.

Cette opération a donc lieu de préférence à une température d'au moins 25°C, plus particulièrement comprise entre 25 et 80°C, de préférence entre 40 et 70°C.

Une fois l'émulsion multiple obtenue, on peut laisser refroidir l'ensemble jusqu'à une température inférieure à la température d'épaississement du polymère thermoépaississant.

Les conditions d'agitation sont de préférence du même type que celles mises en oeuvre lors de la préparation de l'émulsion inverse.

Il est précisé que l'on ne sortirait pas du cadre de la présente invention en mélangeant plusieurs émulsions multiples, dès l'instant que les phases aqueuses externes des émulsions mélangées sont compatibles

L'émulsion selon l'invention peut être utilisée élément constitutif dans des formulations utilisables dans de nombreux domaines, tels que la cosmétique, l'alimentaire, le domaine des matières actives phytosanitaires, celui l'exploitation ou la construction de puits de gisement de pétrole ou de gaz, celui des silicones, ou encore dans le domaine de la fabrication du papier, entre autres.

Un exemple concret mais non limitatif de l'invention va maintenant être présenté.

### EXEMPLE

### 1. Préparation du polymère thermoépaississant

### 1.1/ Synthèse du macromonomère thermosensible

Le macromonomère est synthétisé en masse, sans catalyseur, de la façon suivante :

Dans un réacteur verré bi-cols de 500 ml muni d'un réfrigérant à boules et d'un barreau aimanté, sont introduits à température ambiante l'Antarox E400 (polymère tribloc OE/OP/OE, commercialisé par Rhodia Chimie, 200 g) et l'anhydride maléique (6,74 g).

La température est ensuite portée à 60°C (en environ 1 heure), sous agitation et balayage d'azote, puis à 140°C (en environ 7 heures) et est maintenu à cette température pendant 18 heures.

Le macromonomère obtenu est ensuite solubilisé dans de l'eau et neutralisé en ajoutant de la soude 5M.

Son extrait sec est de 28,9 % (déterminé avec 1 g de solution de macromonomère séché dans une étuve à 105°C pendant 1 heure). Le pH est de 7.

### 1.2/ Synthèse d'un polymère thermoépaississant

Le polymère thermoépaississant est synthétisé par voie radicalaire en solution aqueuse en utilisant de l'acide acrylique et le macromonomère obtenu ci-dessus.

La polymérisation est réalisée environ 45°C.

Le couple réd-ox utilisé est le persulfate d'ammonium (oxydant) et l'acide ascorbique (réducteur).

Les proportions des différents réactifs sont les suivantes :
- Ratio molaire acide acrylique / macromonomère : 98 / 2
- Persulfate d'ammonium : 0,18% molaire par rapport à l'acide acrylique
- Acide ascorbique : 0,09% molaire par rapport à l'acide acrylique

La concentration massique en réactifs est voisine de 16%.

Le procédé est le suivant :

Dans un réacteur verré double enveloppe thermostaté SVL (1,5 I), surmonté d'un réfrigérant tubulaire, équipé d'une agitation mécanique (ancre), muni de plusieurs entrées et d'une arrivée d'azote, on introduit :
A 25°C : le macromonomère en solution aqueuse (68,21 g) et l'eau (139,6 g) ; puis on augmente la température à 45°C.
A 45°C : introduction du persulfate de sodium (0,0316 g) ; ce temps est noté t°

De t° à t°+5h : introduction continue d'acide acrylique (21,01 g) solubilisé dans de l'eau (5,14 g) et partiellement neutralisé par de la soude 5M (4,71 g) ;
De t° à t°+8h : introduction continue de l'acide ascorbique (0,013 g) solubilisé dans de l'eau (15 g).

Le réacteur est ensuite refroidi.

Le polymère obtenu présente les caractéristiques suivantes :

Détermination de l'extrait sec, par gravimétrie (conditions 1.1/) : 16,47 %

Détermination de la masse molaire moyenne, par GPC aqueuse :

### Conditions :

- Eau + 0,5M LiNO₃ + 0,06M NaN₃ à 1 ml/min
- 3 colonnes SHODEX SB 806 M
- Détecteur: Réfractomètre différentiel

Cette GPC est calibrée avec des étalons de POE ; les masses molaires obtenues sont donc des valeurs relatives.

La masse molaire moyenne en masse est de 310000 g/mol.

### Pouvoir thermoépaississant, par rhéologie :

La viscosité de solutions aqueuses contenant le polymère thermoépaississant est mesurée en fonction de la température.

Les conditions de mesure sont les suivantes :

pH=8, extrait sec en polymère de 4 %.

Le saut de viscosité est mesuré en mode écoulement à contrainte imposée en effectuant un balayage de température entre 20°C et 80°C. La configuration utilisée est la géométrie cône-plan 4cm/1degré. La contrainte introduite dans le programme est choisie (en mode manuel) de telle sorte que le gradient à 25°C soit de 10 s⁻¹.

Le saut de viscosité entre 25 et 80°C, correspondant au rapport log₁₀(viscosité à 80°C) / log₁₀(viscosité à 25°C), exprimé en décades, est de 3.

### 2/ Préparation d'une émulsion

### 2.1/ Préparation d'une émulsion inverse

Dans un bécher de 250 ml, on introduit successivement 68,6 g de polybutène Napvis D30, (commercialisé par BP) et le 1,6 g de Tégopren 7006 (polydiméthylsiloxane greffé polyéther polyalkyle, commercialisé par Goldschmidt).

On homogénéise par agitation à la pale cadre à 250 tr/min pendant 10 minutes, la phase organique étant placée dans un bain-marie thermostaté à 70°C.

On introduit ensuite goutte à goutte la phase aqueuse dans les mêmes conditions d'agitation.

La quantité de phase aqueuse est telle que le rapport pondéral phase aqueuse /phase organique est de 30 / 70.

La phase aqueuse comprend 5,2 g d'acide lactique (0,1M), 26,2 g de chlorure de sodium (0,1M) et 0,18 g de Phénonip (biocide, commercialisé par NIPA Laboratories).

Une fois l'introduction de la phase aqueuse réalisée, l'émulsion est affinée par agitation à la pale cadre pendant 10 minutes à 400 tr/min, puis 15 minutes à 600 tr/min.

### 2.2/ Préparation de l'émulsion multiple

La phase aqueuse externe est préparée comme suit :

On mélange 5 g de la solution du polymère thermoépaississant obtenu au point 1.2/, 20 g d'Arlatone F127G à 5% (polymère (OE)x(OP)y(OE)z avec vérification de l'inéquation suivante : 82<x+z<90 et 7 motifs OP pour 1 mole de polymère, commercialisé par ICI), 20 g d'eau distillée.

Le pH initial est ajusté par ajout d'hydroxyde de sodium (2M) pour atteindre un pH de 6-7 (mesuré à 23°C)

La phase aqueuse externe résultante est introduite dans un bain-marie à 50°C sous agitation à la pale TT à 250 tr/min jusqu'à l'obtention d'une phase homogène.

On ajoute ensuite 0,5 g de Phénonip.

On introduit 46,8 g de l'émulsion inverse obtenue précédemment est introduite par ajouts successifs cumulés dans la phase aqueuse externe, à 50°C, sous agitation à 500 tr/min pendant 10 minutes pour chaque ajout.

Une fois l'introduction de l'émulsion inverse terminée, on effectue une opération d'affinage à la pale TT à 700 tr/min pendant 30 minutes.

La proportion pondérale phase aqueuse interne / phase organique *l* phase aqueuse externe : 16 / 35 / 49.

La taille moyenne des gouttelettes est comprise entre 5 et 10 µm (Horiba).

## Revendications

1. Procédé de préparation d'une émulsion huile dans eau, dont la phase organique présente une viscosité supérieure ou égale à 1 Pa.s, dans lequel on met en oeuvre une phase aqueuse comprenant au moins un polymère thermoépaississant présentant un saut de viscosité entre 25 et 80°C tel que la valeur du rapport log₁₀ (viscosité à 80°C) /log₁₀ (viscosité à 25°C) est au moins égale à 1, de préférence au moins égale à 2, la variation de la viscosité étant réversible ; la quantité de polymère thermoépaississant étant telle que la viscosité de la phase aqueuse soit de 0,2 à 5 fois celle de la phase organique à la température de préparation de l'émulsion ; celle-ci étant supérieure ou égale à la température d'épaississement du polymère thermoépaississant.

2. Procédé selon la revendication précédente, **caractérisé en ce que** l'on met en oeuvre un polymère thermoépaississant de structure peigne constitué d'un squelette polymérique sur lequel sont greffés au moins deux segments latéraux polymériques, identiques ou non, et pour lequel soit le squelette polymérique, soit les segments latéraux possèdent une température critique inférieure de solubilité comprise entre 25 et 80°C.

3. Procédé selon l'une des revendications précédentes, **caractérisé en ce que** l'on met en oeuvre un polymère thermoépaississant dont le squelette polymérique du polymère possède une température critique inférieure de solubilité comprise entre 25 et 80°C.

4. Procédé selon l'une des revendications 1 ou 2, **caractérisé en ce que** l'on met en oeuvre un polymère thermoépaississant dont les segments latéraux polymériques du polymère possèdent une température critique inférieure de solubilité comprise entre 25 et 80°C.

5. Procédé selon l'une des revendications précédentes, **caractérisé en ce que** l'on met en oeuvre un polymère comprenant plusieurs polymères agencés entre eux de manière à former une structure réticulée dans laquelle leur segments polymériques possédant la température critique inférieure de solubilité figurent les noeuds de réticulation et au moins une partie de leurs segments ne possédant pas de température critique inférieure de solubilité entre 25 et 80°C établissent les connexions entre lesdits noeuds.

6. Procédé selon l'une des revendications 1 à 3, **caractérisé en ce que** l'on met en oeuvre un polymère thermoépaississant dont les segments ne possédant pas la température critique inférieure de solubilité comprise entre 25 et 80°C, sont hydrosolubles au moins dans ce domaine de température.

7. Procédé selon l'une des revendications précédentes, **caractérisé en ce que** l'on met en oeuvre un polymère thermoépaississant dont les segments polymériques ne possédant pas de température critique inférieure de solubilité sont hydrosolubles dans la plage de température de préparation de l'émulsion.

8. Procédé selon l'une des revendications précédentes, **caractérisé en ce que** l'on met en oeuvre un polymère thermoépaississant dont le segment polymérique ne possédant pas de température critique inférieure de solubilité est un polymère de type éthylénique hydrosoluble.

9. Procédé selon la revendication précédente, **caractérisé en ce que** l'on met en oeuvre un polymère thermoépaississant dérivant de la polymérisation de monomères éthyléniques hydrosolubles de type vinylique, acrylique, styrénique, diénique et/ou de type ester vinylique.

10. Procédé selon l'une des revendications précédentes, **caractérisé en ce que** l'on met en oeuvre un polymère thermoépaississant dont les segments polymériques possèdent un poids moléculaire au moins supérieur à 1000 g/mol.

11. Procédé selon l'une des revendications précédentes, **caractérisé en ce que** l'on met en oeuvre un polymère thermoépaississant dont les segments polymériques sont issus de la polymérisation de l'acide acrylique et/ou l'acide 2-acrylamide-méthyl-propane sulfonique.

12. Procédé selon l'une des revendications précédentes, **caractérisé en ce que** l'on met en oeuvre un polymère thermoépaississant dont les segments polymériques possédant une température inférieur critique de solubilité entre 25 et 80°C dérivent de polymères polyalcoxylés.

13. Procédé selon la revendication précédente, **caractérisé en ce que** l'on met en oeuvre un polymère thermoépaississant dont les segments polymériques possèdent au moins 5 motifs oxyalkylénés.

14. Procédé selon l'une quelconque des revendications précédentes, **caractérisé en ce que** l'on met en oeuvre un polymère thermoépaississant choisi parmi :
- polymère préparé à partir de tri-blocs POE-POP-POE et d'acide acrylique (pourcentages molaires respectifs : 2,3 %, 97,7 %), de préférence par greffage direct,
- polymère préparé à partir de macromonomère de tri-blocs POE-POP-POE et d'acide acrylique (% molaires respectifs : 1,6 %, 98,4 %), de préférence par copolymérisation,
- polymère préparé à partir de macromonomère de tri-blocs POE-POP-POE et d'acide acrylique (% molaires respectifs : 3 %, 97 %), de préférence par copolymérisation, et/ou
- polymère préparé à partir de macromonomère de tri-blocs POE-POP-POE et d'acide acrylique (% molaires respectifs : 2 %, 98 %), de préférence par copolymérisation.

15. Procédé selon l'une quelconque des revendications précédentes, **caractérisé en ce que** l'on met en oeuvre une quantité de polymère thermoépaississant telle que la viscosité de la phase aqueuse soit de 0,5 à 2 fois celle de la phase organique, à la température de préparation de ladite émulsion.

16. Procédé selon l'une des revendications précédentes, **caractérisé en ce que** l'on met en oeuvre une teneur en polymère thermoépaississant comprise entre 0,5 et 5 % en poids de la phase aqueuse, de préférence entre 1 et 3 % en poids de la phase aqueuse.

17. Procédé selon l'une des revendications précédentes, **caractérisé en ce que** l'on met en oeuvre une phase organique présentant une viscosité d'au moins 5 Pa.s, de préférence comprise entre 5 et 500 Pa.s.

18. Procédé selon l'une des revendications précédentes, **caractérisé en ce que** l'on met en oeuvre une phase organique choisie parmi les huiles minérales, les résines alkydes, les polyisocyanates, les silicones de haut poids moléculaire ; ces composés étant seuls ou en mélange.

19. Procédé selon l'une des revendications précédentes, **caractérisé en ce que** l'on met en oeuvre une phase organique comprenant au moins une matière active hydrophobe.

20. Procédé selon l'une des revendications précédentes, **caractérisé en ce que** l'on met en oeuvre une phase organique comprenant une phase aqueuse interne dispersée.

21. Procédé selon la revendication 20, **caractérisé en ce que** l'on met en oeuvre une phase aqueuse interne comprenant au moins une matière active hydrophile.

22. Procédé selon l'une des revendications 20 ou 21, **caractérisé en ce que** l'on met en oeuvre un rapport pondéral phase aqueuse interne / phase organique compris entre 10/90 et 90/10, de préférence, entre 30/70 et 80/20.

23. Procédé selon l'une des revendications précédentes, **caractérisé en ce que** le rapport pondéral phase organique / phase aqueuse, ou le rapport pondéral de l'ensemble phase aqueuse interne et phase organique / phase aqueuse est compris entre 10/90 et 90/10, de préférence compris entre 30/70 et 80/20.

24. Procédé selon l'une des revendications précédentes, **caractérisé en ce que** l'on mélange sous agitation :
+ la phase organique, comprenant :
- éventuellement au moins une matière active hydrophobe,
- éventuellement la phase aqueuse interne dispersée comprenant éventuellement au moins une matière active hydrophile et éventuellement au moins un additif ; l'ensemble phase aqueuse interne et phase organique comprenant au moins un tensioactif non ionique et/ou au moins un polymère amphiphile à blocs, et/ou au moins un tensioactif cationique ; et :
+ la phase aqueuse comprenant :
- éventuellement au moins une matière active hydrophile,
- au moins un tensioactif non ionique polyalcoxylé et/ou au moins un polymère amphiphile non ionique et/ou au moins tensioactif anionique et/ou au moins un polymère amphiphile anionique,
- au moins un polymère thermoépaississant,
- éventuellement au moins un additif et éventuellement au moins un polymère épaississant.

## Patentansprüche

1. Verfahren zur Herstellung einer Öl-in-Wasser-Emulsion, deren organische Phase eine Viskosität kleiner gleich 1 Pa.s aufweist, bei dem man eine wäßrige Phase verwendet, die mindestens ein thermoverdickendes Polymer enthält, welches einen derartigen Viskositätssprung zwischen 25 und 80°C aufweist, daß der Wert des Verhältnisses log₁₀ (Viskosität bei 80°C)/log₁₀ (Viskosität bei 25°C) mindestens gleich 1 und vorzugsweise mindestens gleich 2 ist, wobei die Viskositätsänderung reversibel ist; wobei die Menge an thermoverdickendem Polymer so groß ist, daß die Viskosität der wäßrigen Phase sich auf das 0,2- bis 5fache der Viskosität der organischen Phase bei der Herstellungstemperatur der Emulsion beläuft, wobei diese Temperatur größer gleich der Verdickungstemperatur des thermoverdickenden Polymers ist.

2. Verfahren nach dem vorhergehenden Anspruch, **dadurch gekennzeichnet, daß** man ein thermoverdickendes Polymer mit Kammstruktur verwendet, das aus einer Polymerhauptkette besteht, auf die mindestens zwei gleiche oder verschiedene polymere Seitensegmente aufgepfropft sind, und bei dem entweder die Polymerhauptkette oder die Seitensegmente eine untere kritische Lösungstemperatur zwischen 25 und 80°C aufweisen.

3. Verfahren nach einem der vorhergehenden Ansprüche, **dadurch gekennzeichnet, daß** man ein thermoverdickendes Polymer verwendet, bei dem die Polymerhauptkette des Polymers eine untere kritische Lösungstemperatur zwischen 25 und 80°C aufweist.

4. Verfahren nach Anspruch 1 oder 2, **dadurch gekennzeichnet, daß** man ein thermoverdickendes Polymer verwendet, bei dem die Seitensegmente des Polymers eine untere kritische Lösungstemperatur zwischen 25 und 80°C aufweisen.

5. Verfahren nach einem der vorhergehenden Ansprüche, **dadurch gekennzeichnet, daß** man ein Polymer verwendet, das mehrere Polymere umfaßt, die so miteinander arrangiert sind, daß sie eine vernetzte Struktur bilden, in welcher die polymeren Segmente, die die untere kritische Lösungstemperatur aufweisen, die Vernetzungsknoten bilden und mindestens ein Teil der Segmente, die keine untere kritische Lösungstemperatur zwischen 25 und 80°C aufweisen, die Verbindungen zwischen den Knoten ausbildet.

6. Verfahren nach einem der Ansprüche 1 bis 3, **dadurch gekennzeichnet, daß** man ein thermoverdickendes Polymer verwendet, bei dem die Segmente, die keine untere kritische Lösungstemperatur zwischen 25 und 80°C aufweisen, zumindest in diesem Temperaturbereich wasserlöslich sind.

7. Verfahren nach einem der vorhergehenden Ansprüche, **dadurch gekennzeichnet, daß** man ein thermoverdickendes Polymer verwendet, bei dem die polymeren Segmente, die keine untere kritische Lösungstemperatur aufweisen, im Bereich der Herstellungstemperatur der Emulsion wasserlöslich sind.

8. Verfahren nach einem der vorhergehenden Ansprüche, **dadurch gekennzeichnet, daß** man ein thermoverdickendes Polymer verwendet, bei dem es sich bei dem polymeren Segment, das keine untere kritische Lösungstemperatur aufweist, um ein Polymer vom wasserlöslichen ethylenischen Typ handelt.

9. Verfahren nach einem der vorhergehenden Ansprüche, **dadurch gekennzeichnet, daß** man ein thermoverdickendes Polymer verwendet, das durch Polymerisation von wasserlöslichen ethylenischen Monomeren vom Vinyl-, Acryl-, Styrol-, Dien- und/oder Vinylester-Typ erhältlich ist.

10. Verfahren nach einem der vorhergehenden Ansprüche, **dadurch gekennzeichnet, daß** man ein thermoverdickendes Polymer verwendet, bei dem die polymeren Segmente ein Molekulargewicht von mindestens mehr als 1000 g/mol aufweisen.

11. Verfahren nach einem der vorhergehenden Ansprüche, **dadurch gekennzeichnet, daß** man ein thermoverdickendes Polymer verwendet, bei dem die polymeren Segmente aus der Polymerisation von Acrylsäure und/oder 2-Acrylamidomethylpropansulfonsäure stammen.

12. Verfahren nach einem der vorhergehenden Ansprüche, **dadurch gekennzeichnet, daß** man ein thermoverdickendes Polymer verwendet, bei dem die polymeren Segmente, die eine untere kritische Lösungstemperatur zwischen 25 und 80°C aufweisen, sich von polyalkoxylierten Polymeren ableiten.

13. Verfahren nach dem vorhergehenden Anspruch, **dadurch gekennzeichnet, daß** man ein thermoverdickendes Polymer verwendet, bei dem die polymeren Segmente mindestens 5 oxyalkenylierte Einheiten enthalten.

14. Verfahren nach einem der vorhergehenden Ansprüche, **dadurch gekennzeichnet, daß** man ein unter
- aus POE-POP-POE-Triblöcken und Acrylsäure (jeweilige Molprozentanteile: 2,3%, 97,7%), vorzugsweise durch direkte Pfropfung, hergestelltem Polymer,
- aus POE-POP-POE-Triblock-Makromonomer und Acrylsäure (jeweilige Molprozentanteile: 1,6%, 98,4%), vorzugsweise durch Copolymerisation, hergestelltem Polymer,
- aus POE-POP-POE-Triblock-Makromonomer und Acrylsäure (jeweilige Molprozentanteile: 3%, 97%), vorzugsweise durch Copolymerisation, hergestelltem Polymer, und/oder
- aus POE-POP-POE-Triblock-Makromonomer und Acrylsäure (jeweilige Molprozentanteile: 2%, 98%), vorzugsweise durch Copolymerisation, hergestelltem Polymer
ausgewähltes thermoverdickendes Polymer verwendet.

15. Verfahren nach einem der vorhergehenden Ansprüche, **dadurch gekennzeichnet, daß** man eine derartige Menge an thermoverdickendem Polymer verwendet, daß die Viskosität der wäßrigen Phase das 0,5- bis 2fache der Viskosität der organischen Phase bei der Herstellungstemperatur der Emulsion beträgt.

16. Verfahren nach einem der vorhergehenden Ansprüche, **dadurch gekennzeichnet, daß** man einen Gehalt an thermoverdickendem Polymer zwischen 0,5 und 5 Gew.-%, bezogen auf die wäßrige Phase, und vorzugsweise zwischen 1 und 3 Gew.-%, bezogen auf die wäßrige Phase, verwendet.

17. Verfahren nach einem der vorhergehenden Ansprüche, **dadurch gekennzeichnet, daß** man eine organische Phase mit einer Viskosität von mindestens 5 Pa.s und vorzugsweise zwischen 5 und 500 Pa.s verwendet.

18. Verfahren nach einem der vorhergehenden Ansprüche, **dadurch gekennzeichnet, daß** man eine unter Mineralölen, Alkydharzen, Polyisocyanaten und hochmolekularen Silikonen ausgewählte organische Phase verwendet, wobei diese Verbindungen einzeln oder als Gemisch vorliegen.

19. Verfahren nach einem der vorhergehenden Ansprüche, **dadurch gekennzeichnet, daß** man eine organische Phase mit mindestens einem hydrophoben Wirkstoff verwendet.

20. Verfahren nach einem der vorhergehenden Ansprüche, **dadurch gekennzeichnet, daß** man eine organische Phase mit einer dispergierten internen wäßrigen Phase verwendet.

21. Verfahren nach Anspruch 20, **dadurch gekennzeichnet, daß** man eine interne wäßrige Phase mit mindestens einem hydrophilen Wirkstoff verwendet.

22. Verfahren nach Anspruch 20 oder 21, **dadurch gekennzeichnet, daß** man ein Gewichtsverhältnis von interner wäßriger Phase zu organischer Phase zwischen 10/90 und 90/10 und vorzugsweise zwischen 30/70 und 80/20 verwendet.

23. Verfahren nach einem der vorhergehenden Ansprüche, **dadurch gekennzeichnet, daß** das Gewichtsverhältnis von organischer Phase zu wäßriger Phase oder das Gewichtsverhältnis von interner wäßriger Phase und organischer Phase zusammengenommen zu wäßriger Phase zwischen 10/90 und 90/10 und vorzugsweise zwischen 30/70 und 80/20 liegt.

24. Verfahren nach einem der vorhergehenden Ansprüche, **dadurch gekennzeichnet, daß** man
+ die organische Phase, enthaltend:
- gegebenenfalls mindestens einen hydrophoben Wirkstoff,
- gegebenenfalls die dispergierte interne wäßrige Phase, die gegebenenfalls einen hydrophilen Wirkstoff und gegebenenfalls mindestens ein Additiv enthält; wobei die interne wäßrige Phase und die organische Phase zusammengenommen mindestens ein nichtionisches Tensid und/oder mindestens ein amphiphiles Blockpolymer und/oder mindestens ein kationisches Tensid enthalten; und
+ die wäßrige Phase, enthaltend:
- gegebenenfalls mindestens einen hydrophilen Wirkstoff,
- mindestens ein polyalkoxyliertes nichtionisches Tensid und/oder mindestens ein nichtionisches amphiphiles Polymer und/oder mindestens ein anionisches Tensid und/oder mindestens ein anionisches amphiphiles Polymer,
- mindestens ein thermoverdickendes Polymer,
- gegebenenfalls mindestens ein Additiv und gegebenenfalls mindestens ein verdickendes Polymer;
unter Rühren vermischt.

## Claims

1. Process for preparing an oil-in-water emulsion the organic phase of which has a viscosity of greater than or equal to 1 Pa.s, in which an aqueous phase is used comprising at least one heat-induced thickening polymer displaying a jump in viscosity between 25 and 80°C such that the value of the ratio log₁₀ (viscosity at 80°C)/log₁₀ (viscosity at 25°C) is at least equal to 1 and preferably at least equal to 2, the variation in viscosity being reversible; the amount of heat-induced thickening polymer being such that the viscosity of the aqueous phase is from 0.2 to 5 times that of the organic phase at the emulsion preparation temperature; said temperature being greater than or equal to the thickening temperature of the heat-induced thickening polymer.

2. Process according to the preceding claim, **characterized in that** a heat-induced thickening polymer of comb structure is used, consisting of a polymer skeleton onto which are grafted at least two identical or different polymeric side segments, and for which either the polymer skeleton or the side segments have a lower critical solution temperature of between 25 and 80°C.

3. Process according to either of the preceding claims, **characterized in that** a heat-induced thickening polymer for which the polymer skeleton of the polymer has a lower critical solution temperature of between 25 and 80°C is used.

4. Process according to either of Claims 1 and 2, **characterized in that** a heat-induced thickening polymer for which the polymeric side segments of the polymer have a lower critical solution temperature of between 25 and 80°C is used.

5. Process according to one of the preceding claims, **characterized in that** a polymer is used comprising several polymers arranged together so as to form a crosslinked structure in which the polymer segments thereof having the lower critical solution temperature contain the crosslinking nodes and at least some of the segments thereof not having a lower critical solution temperature of between 25 and 80°C establish the connections between said nodes.

6. Process according to one of Claims 1 to 3, **characterized in that** a heat-induced thickening polymer is used whose segments not having a lower critical solution temperature of between 25 and 80°C are water-soluble at least in this temperature range.

7. Process according to one of the preceding claims, **characterized in that** a heat-induced thickening polymer is used, the polymer segments of which not having a lower critical solution temperature are water-soluble in the temperature range for preparation of the emulsion.

8. Process according to one of the preceding claims, **characterized in that** a heat-induced thickening polymer is used whose polymer segment not having a lower critical solution temperature is a polymer of water-soluble ethylenic type.

9. Process according to the preceding claim, **characterized in that** a heat-induced thickening polymer derived from the polymerization of water-soluble ethylenic monomers of vinyl, acrylic, styrene or diene type and/or of vinyl ester type is used.

10. Process according to one of the preceding claims, **characterized in that** a heat-induced thickening polymer whose polymer segments have a molecular weight at least greater than 1 000 g/mol is used.

11. Process according to one of the preceding claims, **characterized in that** a heat-induced thickening polymer whose polymer segments are derived from the polymerization of acrylic acid and/or 2-acrylamidomethylpropanesulfonic acid is used.

12. Process according to one of the preceding claims, **characterized in that** a heat-induced thickening polymer the polymer segments of which having a lower critical solution temperature of between 25 and 80°C are derived from polyalkoxylated polymers is used.

13. Process according to the preceding claim, **characterized in that** a heat-induced thickening polymer whose polymer segments contain at least 5 oxyalkylenated units is used.

14. Process according to any one of the preceding claims, **characterized in that** a heat-induced thickening polymer chosen from the following is used:
- polymer prepared from PEO-PPO-PEO triblocks and from acrylic acid (respective molar percentages: 2.3%, 97.7%), preferably by direct grafting,
- polymer prepared from PEO-PPO-PEO triblock macro-monomer and from acrylic acid (respective molar percentages: 1.6%, 98.4%), preferably by copolymerization,
- polymer prepared from PEO-PPO-PEO triblock macro-monomer and from acrylic acid (respective molar percentages: 3%, 97%), preferably by copolymerization, and/or
- polymer prepared from PEO-PPO-PEO triblock macro-monomer and from acrylic acid (respective molar percentages: 2%, 98%), preferably by copolymerization.

15. Process according to any one of the preceding claims, **characterized in that** an amount of heat-induced thickening polymer is used such that the viscosity of the aqueous phase is from 0.5 to 2 times that of the organic phase, at the preparation temperature of said emulsion.

16. Process according to one of the preceding claims, **characterized in that** a content of heat-induced thickening polymer of between 0.5% and 5% by weight of the aqueous phase and preferably between 1% and 3% by weight of the aqueous phase is used.

17. Process according to one of the preceding claims, **characterized in that** an organic phase with a viscosity of at least 5 Pa.s and preferably between 5 and 500 Pa.s is used.

18. Process according to one of the preceding claims, **characterized in that** an organic phase chosen from mineral oils, alkyd resins, polyisocyanates and high molecular weight silicones is used; these compounds being alone or as a mixture.

19. Process according to one of the preceding claims, **characterized in that** an organic phase comprising at least one hydrophobic active material is used.

20. Process according to one of the preceding claims, **characterized in that** an organic phase comprising a dispersed internal aqueous phase is used.

21. Process according to Claim 20, **characterized in that** an internal aqueous phase comprising at least one hydrophilic active material is used.

22. Process according to either of Claims 20 and 21, **characterized in that** an internal aqueous phase/organic phase weight ratio of between 10/90 and 90/10 and preferably between 30/70 and 80/20 is used.

23. Process according to one of the preceding claims, **characterized in that** the organic phase/aqueous phase weight ratio, or the weight ratio of the internal aqueous phase and organic phase combination/aqueous phase, is between 10/90 and 90/10 and preferably between 30/70 and 80/20.

24. Process according to one of the preceding claims, **characterized in that** the following are mixed with stirring:
+ the organic phase comprising:
- optionally at least one hydrophobic active material,
- optionally the dispersed internal aqueous phase optionally comprising at least one hydrophilic active material and optionally at least one additive; the combination of internal aqueous phase and organic phase comprising at least one nonionic surfactant and/or at least one amphiphilic block polymer, and/or at least one cationic surfactant; and
+ the aqueous phase comprising:
- optionally at least one hydrophilic active material,
- at least one polyalkoxylated nonionic surfactant and/or at least one nonionic amphiphilic polymer and/or at least one anionic surfactant and/or at least one anionic amphiphilic polymer,
- at least one heat-induced thickening polymer,
- optionally at least one additive and optionally at least one thickening polymer.
